# Europäisches Patentamt

# European Patent Office

# Office européen des brevets

(19)

(11) Veröffentlichungsnummer: **0 060 528**
**A1**

(12) **EUROPÄISCHE PATENTANMELDUNG**

(21) Anmeldenummer: **82101983.3**

(22) Anmeldetag: **12.03.82**

(51) Int. Cl.³: **C 07 C 17/16, C 07 C 19/02**

(30) Priorität: **16.03.81 DE 3110165**

(43) Veröffentlichungstag der Anmeldung: **22.09.82**
**Patentblatt 82/38**

(84) Benannte Vertragsstaaten: **BE DE FR GB IT NL**

(71) Anmelder: **HOECHST AKTIENGESELLSCHAFT,**
**Postfach 80 03 20, D-6230 Frankfurt/Main 80 (DE)**

(72) Erfinder: **Grünbein, Wolfgang, Dr.,**
**Alt-Oberliederbach 35, D-6237 Liederbach (DE)**
Erfinder: **Lendle, Wilhelm, Dr., Hirschpfad 5, D-6232 Bad**
**Soden am Taunus (DE)**
Erfinder: **Post, Hendrik Willem, Paulinenweg 4,**
**D-6238 Hofheim am Taunus (DE)**
Erfinder: **Richter, Heinz, Wilhelm-Bonn-Strasse 2,**
**D-6242 Kronberg/Taunus (DE)**
Erfinder: **Rossberg, Manfred, Dr., Am Rotfeld 8,**
**D-6273 Waldems (DE)**

(54) **Verfahren zur Gewinnung von Methylchlorid enthaltenden Gasen.**

(57) Methylchlorid enthaltende Gase werden aus Chlorwasserstoff enthaltenden Gasen und Methanol an einem mit Zinksalzen dotierten $Al_2O_3$-Katalysator bei erhöhter Temperatur genommen. Als Chlorwasserstoff enthaltendes Gas wird ein Gemisch der Zusammensetzung

| | |
|---|---|
| $CH_3Cl + CH_4$ | 15 - 80 Vol.-% |
| $N_2$ | 3 - 50 Vol.-% |
| HCl | 10 - 25 Vol.-% |
| $CCl_3H + CCl_4$ | 0,1 - 5 Vol.-% |
| $CH_2Cl_2$ | 5 - 20 Vol.-% |
| $Cl_2$ | 0 -0,1 Vol.-% |

eingesetzt.

HOECHST AKTIENGESELLSCHAFT    HOE 81/F 052    Dr.SP/cr

0060528

## Verfahren zur Gewinnung von Methylchlorid enthaltenden Gasen

Die vorliegende Erfindung betrifft ein Verfahren zur Herstellung von Methylchlorid enthaltenden Gasen aus Chlorwasserstoff enthaltenden Gasen und Methanol in der Gasphase an einem festen Aluminiumoxid-Kontakt.

Bei der technischen Chlorierung von Methan fällt neben den Chlorierungsprodukten, wie Methylchlorid oder Methylenchlorid, noch Chlorwasserstoff an, der aufgearbeitet werden muß. Das gleiche gilt für die Chlorierung von Methylchlorid, Methylenchlorid oder Chloroform. Neben der Elektrolyse von wäßrigem Chlorwasserstoff unter Rückgewinnung von Chlor ist in diesem Zusammenhang vor allem die Reaktion des Chlorwasserstoffs mit Methanol von Interesse, da hierbei ohne Einsatz von Chlor und Methan ein technisches Methanchlorierungsprodukt erhalten werden kann.

Die Umsetzung von Chlorwasserstoff und Methanol, die sogenannte "Methanolveresterung", kann unter anderem an einem festen Katalysator auf Basis von aktiviertem $Al_2O_3$ bei Temperaturen von 250 bis 350°C und einem Druck von etwa 5 bar erfolgen (Ullmanns Enzyclopädie der Technischen Chemie, 3. Auflage, 1954, Band 5, Seite 401). Für diese Umsetzung wird in US-PS 18 39 089 ein $Al_2O_3$-Katalysator empfohlen, der mit Zinkchlorid aktiviert wurde.

Ungünstigerweise ist der bei der Methanchlorierung anfallende Chlorwasserstoff nicht rein, sondern enthält mindestens äquimolare Mengen an Methanchlorierungsprodukten. In manchen Fällen wird die Chlorierung in Gegenwart eines Inertgases, wie z.B. Stickstoff, durchgeführt, was zu einer weiteren Verringerung des Chlorwasserstoff-Gehalts der Reaktionsgase führt.

- 2 -    0060528

Die Abtrennung des Chlorwasserstoffs aus diesem Gemisch geschieht in der Technik meist durch Waschen mit Wasser und Austreiben von HCl aus der wäßrigen Absorptionslösung durch Erhitzen. Ferner kann man aus den Reaktionsprodukten der Methan- oder Methylchlorid-Chlorierung durch Auswaschen mit Chlormethanen und nachfolgende Verflüssigung Chlorwasserstoff gewinnen oder durch direkte Verflüssigung und fraktionierte Destillation unter Druck einen relativ reinen trockenen Chlorwasserstoff erhalten. Dieser Chlorwasserstoff kann zur Methanolveresterung eingesetzt werden. Beispielsweise kann gemäß DE-OS 18 06 988 ein Gasstrom, der aus 87 Mol.-% HCl und 13 Mol-% Methylchlorid besteht, mit Methanol vermischt und in eine Methanolveresterungs-Vorrichtung eingeführt werden, die einen Katalysator aus aktivierter Tonerde enthält.

Es ist ein Nachteil dieses Verfahrens, daß der Methanolveresterung Trennoperationen vorgeschaltet werden müssen. Dies gilt umso mehr, wenn die Chlorierung von Methan (oder Methylchlorid) in Gegenwart von Inertgasen stattfindet.

Es ist ferner ein Nachteil der bekannten Gasphasen-Reaktion, daß der Katalysator relativ schnell inaktiv wird. Dieser Vorgang wird von einer Kohlenstoff-Abscheidung begleitet (Chemie-Ingenieur-Technik Band $\underline{42}$ (1970), S. 1215-1219). Dort wird auch angegeben, daß die Reaktionsgeschwindigkeit durch die Endprodukte nicht beeinflußt wird und das Reaktionsgleichgewicht ganz auf Seiten der Endprodukte liegt. Die Inaktivierung zeigt sich darin, daß am Reaktorausgang steigende Mengen an nicht umgesetztem Methanol anfallen. Wesentlich beschleunigt wird die Inaktivierung, wenn mehrfach chlorierte $C_1$- und $C_2$-Kohlenwasserstoffe, wie Tetrachlorkohlenstoff oder Chloroform bei der Umsetzung zugegen sind.

Es bestand daher die Aufgabe, ein Verfahren zu finden, bei dem der Chlorwasserstoff der Methan- oder Methylchlorid-Chlorierung in Gegenwart von Stickstoff ohne vorherige Abtrennung für die Methanolveresterung eingesetzt werden kann.

Die vorliegende Erfindung löst diese Aufgabe.

Es wurde nun ein Verfahren zur Herstellung von Methylchlorid enthaltenden Gasen aus Chlorwasserstoff enthaltenden Gasen und Methanol gefunden, das an einem mit Zinksalzen dotierten $Al_2O_3$-Katalysator bei erhöhter Temperatur abläuft. Das Verfahren ist dadurch gekennzeichnet, daß man als Chlorwasserstoff enthaltendes Gas ein Gemisch der Zusammensetzung

| | |
|---|---|
| $CH_3Cl + CH_4$ | 15 und/oder 80 Vol.-% |
| $N_2$ | 3 - 50 Vol.-% |
| HCl | 10 - 25 Vol.-% |
| $CCl_3H + CCl_4$ | 0,1 und/oder 5 Vol.-% |
| $CH_2Cl_2$ | 5 - 10 Vol.-% |
| $Cl_2$ | 0 - 0,1 Vol.-%. |

einsetzt.

Die Herstellung der eingesetzten Katalysatoren ist an sich bekannt. Man kann beispielsweise einen wäßrigen Brei, der aus Aluminiumoxid und Zinkoxid besteht, zu Granulaten verpressen und trocknen oder Aluminiumoxid-Granulate mit Lösungen von Zinkchlorid tränken und trocknen. Im allgemeinen enthalten die Katalysatoren 1 bis 50 Gew.-% Zink.

Je nach der Konzentration an Chlorwasserstoff und Zink springt die Reaktion bei Temperaturen von 150 bis 180°C an. Die Arbeitstemperaturen liegen bei 150 bis 350°C. Bevorzugt sind Temperaturen von 150 bis 245°C. Bei Temperaturen von 250 bis 350°C verläuft die Reaktion

gleichfalls; jedoch kommt es zu immer stärkerer Abscheidung von Kohlenstoff. Da sich während der Reaktion das Volumen eines Ansatzes nicht ändert, ist der angewandte Druck nicht kritisch. Im allgemeinen wird bei Drucken von 1 bis 10 bar gearbeitet.

Das Molverhältnis Methanol / Chlorwasserstoff beträgt 0,2 : 1 bis 2:1, vorzugsweise 0,3:1 bis 0,9 : 1. Der Chlorgehalt des eingesetzten Gases beträgt vorzugsweise 0,01 - 0,05 Vol.-%, der Gehalt an Methylchlorid vorzugsweise 50 - 80 Vol.-%.

Nach dem erfindungsgemäßen Verfahren läßt sich der Chlorwasserstoff, der bei der Kreislaufchlorierung von Methan- oder Methylchlorid anfällt, weiterverarbeiten. Im Reaktionsprodukt werden, nach Entfernung des Wassers durch Schwefelsäure-Trocknung, Methylenchlorid und höhere Chlorierungsprodukte zusammen oder getrennt auskondensiert. Das Restgas wird nach Trocknen wieder in den Chlorierungsreaktor zurückgeleitet. Falls gewünscht, kann Methylchlorid durch Verflüssigung oder durch Auswaschen mit Tetrachlorkohlenstoff bei tiefer Temperatur abgetrennt und durch fraktionierte Destillation rein erhalten werden.

Es ist ein Vorteil des vorliegenden Verfahrens, daß wegen der hohen Anteile an Inertgas nur eine geringe Wärmemenge bei der Reaktion freigesetzt wird und sich daher in der Katalysatorschicht keine besonders heiße Zone ("hot spot") ausbildet, die erfahrungsgemäß zu einer schnellen Katalysator-Desaktivierung führt.

Die Erfindung wird durch das Beispiel näher erläutert.

Beispiel

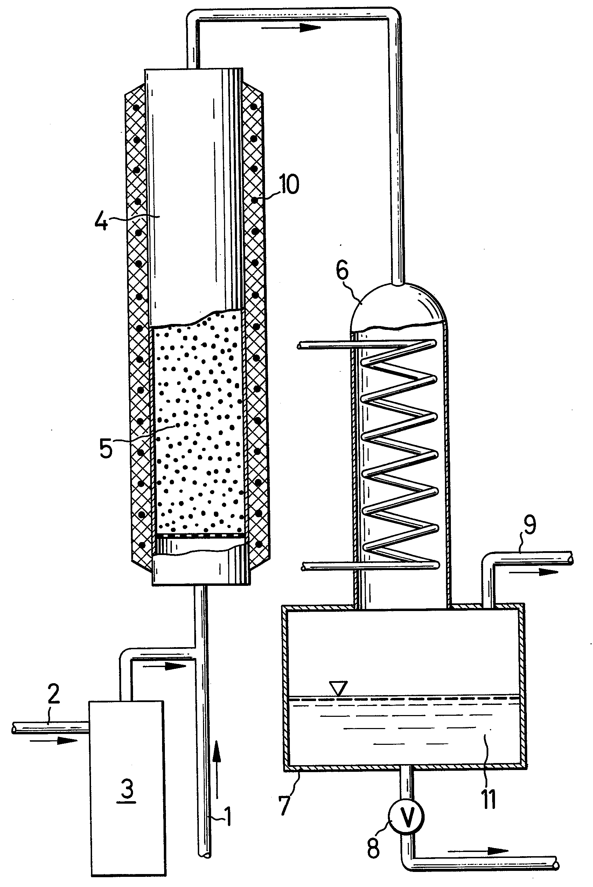

Die Versuchsapparatur wird in der Figur dargestellt.
Die Reaktion findet statt in einem Nickelrohr (Innendurchmesser 50 mm, Länge 70 cm), das von außen durch
den Ofen (10) elektrisch beheizt wird. Das Chlorwasserstoff enthaltende Gas wird über Leitung (1) dem
Reaktor zugeführt, der aus Nickelrohr (4), Ofen (10) und
Kontaktfüllung (5) besteht. Über Leitung (2) wird
flüssiges Methanol in den Verdampfer (3) eingeleitet,
dort verdampft und schließlich dem Chlorwasserstoff
enthaltenden Gas zugemischt.

Es wird ein käuflicher $Al_2O_3$-Katalysator verwendet
(Harshaw Al 3996), der mit 10 Gew.-% Zinkchlorid
dotiert wurde.

Nach der Reaktion gelangen die Gase in den Kondensator
(6) in dem das Wasser niedergeschlagen wird, das bei
der Methanolveresterung entsteht. Das Kondensat (11)
enthält ferner Restmengen an Chlorwasserstoff und
Methanol, die nicht umgesetzt wurden. Das Kondensat (11)
sammelt sich im Behälter (7) an und kann durch den Hahn
(8) abgelassen werden. Die nicht-kondensierten Reaktionsgase verlassen das System über Leitung (9). Das in
(7) anfallende Kondensat kann mit Vorteil einer Methanolhydrochlorierungsanlage zugeführt werden.

Dem Reaktor werden pro Stunde 316 l eines Gases zugeführt, das bei der Chlorierung von Methylchlorid mit
Chlorunterschuß gewonnen wurde und folgende Zusammensetzung aufweist (Meßgenauigkeit 5 %):

27.3 Vol.-% $CH_3Cl$
10.4 Vol.-% $CH_2Cl_2$
2.4 Vol.-% $CHCl_3$
0.2 Vol.-% $C Cl_4$
15.5 Vol.-% $HCl$
Rest $N_2$

In den Reaktor wurden ferner 28 g Methanol dampfförmig eingeleitet. Die Reaktionstemperatur betrug in der Mitte des Katalysators 221°C. Man erhält bei der Kondensation 25 g wäßriges Kondensat , das 31,2 Gew.-% HCl und 6,1 Gew.-% Methanol enthält. Das Restgas hat folgende Zusammensetzung (Meßgenauigkeit 5 %):

33.7 Vol.-% $CH_3Cl$
10.4 Vol.-% $CH_2Cl_2$
2.4 Vol.-% $CHCl_3$
0.2 Vol.-% $C Cl_4$
7.7 Vol.-% $HCl$
Rest $N_2$

PATENTANSPRUCH

Verfahren zur Herstellung von Methylchlorid enthaltenden Gasen aus Chlorwasserstoff enthaltenden Gasen und Methanol durch Reaktion an einem mit Zinksalzen dotierten $Al_2O_3$-Katalysator bei erhöhter Temperatur, dadurch gekennzeichnet, daß man als Chlorwasserstoff enthaltendes Gas ein Gemisch der Zusammensetzung

$CH_3Cl$ und/oder $CH_4$  15 - 80 Vol.-%

$N_2$  3 - 50 Vol.-%

HCl  10 - 25 Vol.-%

$CCl_3H$ und/oder $CCl_4$  0,1 - 5 Vol.-%

$CH_2Cl_2$  5 - 20 Vol.-%

$Cl_2$  0 - 0,1 Vol.-%

einsetzt.

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | <u>DE - B2 - 1 907 088</u>  (THE DOW CHE-MICAL CO.) <br><br> + Anspruch 1 + <br><br> -- | 1 | C 07 C 17/16 <br> C 07 C 19/02 |
| A | <u>DE - B - 1 543 063</u> (IMPERIAL CHE-MICAL INDUSTRIES LTD) <br><br> + Anspruch 1 + <br><br> -- | 1 | |
| A | <u>GB - A - 1 256 708</u> (STAUFFER CHEM-ICAL COMPANY) <br><br> + Anspruch 1 + <br><br> ---- | 1 | |

**RECHERCHIERTE SACHGEBIETE (Int. Cl.³)**

C 07 C 17/00

C 07 C 19/00

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X   Der vorliegende Recherchenbericht wurde für alle Patentansprüche erstellt.

| Recherchenort | Abschlußdatum der Recherche | Prüfer |
|---|---|---|
| WIEN | 21-05-1982 | REIF |

EPA form 1503.1   06.78